# EUROPÄISCHE PATENTSCHRIFT

(11) **EP 0 695 700 B1**
(45) Veröffentlichungstag und Bekanntmachung des Hinweises auf die Patenterteilung: **19.08.1998**
(21) Anmeldenummer: 95104029.4
(22) Anmeldetag: 18.03.1995
(51) Int. Cl.: B65D 83/76, B65D 35/30, A61M 35/00, B29C 49/20

(54) **Behältnis für fliessfähige, insbesondere pasteuse Stoffe und Verfahren zu seiner Herstellung**
Container for flowable, in particular pasty substances and its manufacturing process
Récipient pour substances pouvant s'écouler, en particulier pâteuses et son procédé de fabrication

(30) Priorität: 14.06.1994 DE 4420594
(43) Veröffentlichungstag der Anmeldung: 07.02.1996
(73) Patentinhaber: Hansen, Bernd, Dipl.-Ing., D-74429 Sulzbach-Laufen (DE)
(72) Erfinder: Hansen, Bernd, Dipl.-Ing., D-74429 Sulzbach-Laufen (DE)
(74) Vertreter: Patentanwälte Bartels und Partner

(56) Entgegenhaltungen:
- EP-A- 0 099 706
- BE-A- 568 148
- DE-A- 3 202 275
- FR-A- 552 617
- FR-E- 83 278
- US-A- 3 297 207
- US-A- 3 493 147
- US-A- 4 319 701

## Beschreibung

Die Erfindung betrifft ein Behältnis aus Kunststoff für eine dosierbare Abgabe von fließfähigen, insbesondere pasteusen Stoffen, das die Merkmale des Oberbegriffs des Anspruches 1 aufweist. Ferner betrifft die Erfindung ein Verfahren zur Herstellung eines derartigen Behältnisses.

Bekannte vergleichbare Behältnisse dienen der Aufnahme und dosierbaren Abgabe von Salben und sind als Ampullen ausgebildet, wobei an das der Abgabeöffnung abgewandte Ende ein luftgefüllter Balg angeformt ist. Für die Abgabe der Salbe wird die Ausgabeöffnung der Ampulle geöffnet und der Balg zusammengedrückt, damit die aus dem Balg austretende Luft die Salbe aus der Ampulle verdrängt. Dabei kann es jedoch dazu kommen, daß sich durch die Salbe hindurch oder längs der Innenwand der Ampulle ein Luftkanal bildet, was zur Folge hat, daß ein mehr oder weniger großer Teil des Inhalts der Ampulle aus dieser nicht herausgedrückt wird. Dies ist sowohl wegen der dadurch stark variierenden Abgabemenge, also der ungenauen Dosierung, als auch vor allem bei teuren Produkten wegen der verlorenen Restmenge störend.

Durch die US-PS 3 297 207 ist ein gattungsgemäßes Behältnis aus Kunststoff bekannt. Das bekannte Behältnis besteht aus einer Tube mit einem üblichen Schraubverschluß. Durch Zusammenpressen der Tubenwände hinter dem Trennkolben wird dieser in mehreren Schritten in Richtung der Ausgabeöffnung bewegt, um so portionsweise den bevorrateten pasteusen Stoff abzugeben. Dieses bekannte Behältnis ist auch teuer in der Herstellung.

Ausgehend von diesem Stand der Technik liegt der Erfindung die Aufgabe zugrunde, ein Behältnis zu schaffen, bei dem sichergestellt ist, daß sein Inhalt nahezu vollständig durch die Ausgabeöffnung hindurch ausgegeben werden kann, das aber dennoch kostengünstig ist. Diese Aufgabe löst ein Behältnis mit den Merkmalen des Anspruches 1.

Dadurch, daß gemäß dem kennzeichnenden Teil des Anspruches 1 die Ausgabeöffnung des Behältnisses mittels eines Brechverschlusses verschlossen ist und daß die Kammer blockförmig ausgebildet und von dünnen Wänden begrenzt oder aus einem Balg gebildet ist, bleibt von dem im Behältnis enthaltenen Stoff allenfalls ein an seiner Innenwand haftender dünner Film zurück. Außerdem können das die Ausgabeöffnung bildende Ende des Behälters und die ihr zugewandte Stirnseite des Trennkolbens so ausgebildet sein, daß auch hier keine nennenswerte Restmenge zurückbleibt, wobei durch einmaliges Zusammendrücken der Kammer die in ihr bevorratete Luft den Trennkolben bis zur Ausgabeöffnung des Zylinders bewegt und den im Behältnis bevorrateten Stoff vollständig über die Ausgabeöffnung abgeben kann. Ein mehrfaches Zusammenpressen des Behältnisses ist mithin nicht notwendig und die erreichbare Dosiergenauigkeit ist sehr hoch. Ferner läßt sich das erfindungsgemäße Behältnis sehr kostengünstig herstellen, zumal es nur aus zwei Teilen, nämlich dem Behältnis und der Kammer einerseits sowie dem Trennkolben andererseits zu bestehen braucht. Der Fertigungsaufwand kann deshalb sehr gering gehalten werden, so daß das erfindungsgemäße Behältnis wesentlich kostengünstiger ist als beispielsweise eine Einmalspritze entsprechender Größe.

Bei dem Trennkolben kann es sich um ein aus Kunststoff bestehendes Einlegeteil handeln. Als Kunststoff kommt dabei der gleiche Kunststoff in Frage, aus dem das Behältnis besteht. Dies hat den Vorteil, daß der im Behältnis enthaltene Stoff nicht mit unterschiedlichen Materialien in Berührung kommt. Besonders vorteilhaft ist Polyfluorethylen, da es inert ist. Selbstverständlich kommen aber auch Polyethylene und Polypropylene in Frage.

Zwar wird man vorteilhafterweise die Außenkontur des Trennkolbens an die vorzugsweise kreiszylindrische Innenkontur des Zylinders weitgehend anpassen. Da jedoch vermieden werden muß, daß bei einer ungünstigen Toleranzsituation der Kolben schwergängig ist, ist bei einer bevorzugten Ausführungsform an den Trennkolben wenigstens eine in sich geschlossene Dichtlippe angeformt, welche mit leichtem Druck an der Zylinderwand anliegt. Selbstverständlich kommen auch andere Dichtungsmittel, beispielsweise ein O-Ring, in Frage. An den Trennkolben angeformte Dichtungsmittel wie die genannte Dichtlippe sind aber aus Kostengründen vorteilhafter. Ob auf Dichtungsmittel ganz verzichtet werden kann, hängt in erster Linie von den Eigenschaften des mittels des Trennkolbens zu verdrängenden Stoffes ab.

Die an das eine Ende des Zylinders angeformte Kammer kann als ein sich in Zylinderlängsrichtung erstreckender Faltenbalg ausgebildet sein. Wenn ein solcher Faltenbalg vorgesehen ist, kann der Trennkolben am einen Ende einer Kolbenstange angeordnet sein, welche in den Faltenbalg ragt und gegen die Ausgabeöffnung hinverschoben werden kann, wenn man den Faltenbalg in axialer Richtung zusammendrückt.

Die Kammer kann aber auch als ein quer zur Längsrichtung des Behältnisses zusammendrückbarer Balg ausgebildet sein. Der Trennkolben wird dann mit Hilfe der Luft im Zylinder verschoben, welche aus dem Balg herausgedrückt werden kann. Selbstverständlich muß in diesem Falle das Volumen des Balges ausreichend groß sein.

Vorzugsweise ist der Behälter an dem die Ausgangsöffnung bildenden Ende mittels eines Brechverschlusses verschlossen, der längs einer Sollbruchstelle durch ein Kippen oder Drehen relativ zum Behälterkörper von diesem abgetrennt werden kann.

Der Erfindung liegt auch die Aufgabe zugrunde, ein vorteilhaftes Verfahren zur Herstellung des erfindungsgemäßen Behältnisses anzugeben. Diese Aufgabe löst ein Verfahren mit den Merkmalen des Anspruches 10. Mit diesem Verfahren wird das Behältnis einschließlich seines Brechverschlusses und der Kammer, vorzugsweise aus einem extrudierten Schlauchabschnitt, hergestellt, in der Maschine gefüllt, mit dem Trennkolben ergänzt und dann verschlossen. Alle mit dem eingefüllten Stoff in Berührung kommenden Flächen sind ebenso wie die in der Kammer eingeschlossene, sterilisierte Luft antiseptisch, weil die für ein Blasformen erforderliche Temperatur ausreichend hoch ist. Die Herstellung des Trennkolbens erfolgt vorteilhafterweise gemäß Anspruch 11.

Im folgenden ist die Erfindung anhand von zwei in der Zeichnung dargestellten Ausführungsbeispielen im einzelnen erläutert. Es zeigen
- Fig. 1: eine aufgebrochen dargestellte Ansicht eines ersten Ausführungsbeispiels,
- Fig. 2: einen vergrößert dargestellten Ausschnitt aus Fig.1,
- Fig. 3: eine aufgebrochen dargestellte Ansicht eines zweiten Ausführungsbeispiels,
- Fig. 4: einen vergrößert dargestellten Ausschnitt aus Fig.3.

Eine aus Kunststoff bestehende Ampulle für eine dosierbare Abgabe eines in ihr enthaltenen, fließfähigen Stoffen, beispielsweise einer pharmazeutischen oder kosmetischen Salbe, weist einen kreiszylindrischen Ampullenkörper 1 auf, der an seinem einen Ende halbkugelartig ausgebildet ist und hier eine kreisrunde Ausgabeöffnung aufweist, die mittels eines Brechverschlusses 2 verschlossen ist. Der Brechverschluß 2 ist, um die Brechkraft bequem aufbringen zu können, mit einem plattenförmigen Knebel 3 versehen, der mit zwei Fingern erfaßt werden kann. Die Trennung des Brechverschlusses 2 vom Ampullenkörper 1 erfolgt längs einer Sollbruchstelle 4 verringerter Wandstärke, die längs des Randes der kreisförmigen Ausgabeöffnung verläuft, so daß nach dem Entfernen des Brechverschlusses 2 die Ausgabeöffnung freiliegt und ein zentral angeordnetes Loch in dem halbkugelförmigen Endstück des Ampullenkörpers 1 bildet.

An das andere Ende des Ampullenkörpers 1 ist einstückig eine blockförmige Kammer 5 angeformt, die sich in Längsrichtung des Ampullenkörpers 1 erstreckt und von so dünnen Wänden begrenzt ist, daß sie mit zwei Fingern zusammengedrückt werden kann. Ihr Volumen ist mindestens so groß wie das Volumen des aus der Ampulle zu verdrängenden Stoffes.

Der in den Ampullenkörper 1 eingefüllte Stoff ist vom Innenraum der Kammer 5 und der in ihr enthaltenen Luft durch einen Trennkolben 6 getrennt, der längsverschiebbar im Ampullenkörper 1 angeordnet ist und mit seiner Mantelfläche an der Innenmantelfläche des Ampullenkörpers 1 anliegt oder aus Toleranzgründen vorzugsweise im Durchmesser etwa 0,4mm bis 0,8mm kleiner ist als der Innendurchmesser des Ampullenkörpers 1. Der Trennkolben 6 hat deshalb eine kreiszylindrische Querschnittsform. An dem gegen die Ausgabeöffnung weisenden Ende ist der Übergang von der Mantelfläche zur Stirnfläche abgerundet, damit der Trennkolben 6 in den halbkugelartigen Endabschnitt des Ampullenkörpers 1 eintauchen und auch hier den im Ampullenkörper 1 enthaltenen Stoff nahezu vollständig aus der Ausgabeöffnung herausdrücken kann.

Die Ampulle besteht also nur aus zwei Teilen, die vorzugsweise aus demselben Kunststoff, beispielsweise Polyethylen oder Polypropylen, hergestellt sind. Für den Trennkolben 6 kommt aber auch das unter der Handelsbezeichnung Teflon bekannte Polyfluorethylen in Frage, da dieses Material inert ist.

Nachdem der Brechverschluß 2 entfernt ist, wird die Kammer 5 zusammengedrückt, also im Sinne einer Verringerung ihres Volumens deformiert. Die Wände der Kammer 5 sind so dimensioniert, daß die hierfür erforderliche Kraft quer zur Kammerlängsrichtung ohne Schwierigkeiten mit zwei Fingern aufgebracht werden kann. Die aus der Kammer 5 verdrängte Luft drückt den Trennkolben 6 gegen die Ausgabeöffnung des Ampullenkörpers 1. Dabei schiebt der Trennkolben 6 den im Ampullenkörper 1 enthaltenen Stoff vor sich her. Es bleibt allenfalls ein dünner Film an der Innenfläche des Ampullenkörpers 1 haften. Das Volumen der Kammer 5 ist so groß gewählt, daß der Trennkolben 6 bis zur Anlage an dem halbkugelartigen Ende des Ampullenkörpers 1 bewegt werden kann, so daß nur eine sehr geringe Restmenge des Stoffes im Ampullenkörper 1 zurückbleibt.

Die Herstellung, Füllung und das Verschließen der Ampulle erfolgt in einer Blasformmaschine. Dort wird zunächst der Ampullenkörper 1 und der Brechverschluß 2 durch Blasformen geformt. Dann wird der Ampullenkörper 1 mit einer vorbestimmten Menge des in der Ampulle aufzubewahrenden Stoffes gefüllt. Danach wird der Trennkolben 6 in den Ampullenkörper 1 maschinell eingesetzt und zum Schluß wird durch das Anformen der Kammer 5 der Ampullenkörper 1 dicht verschlossen.

Vorzugsweise werden die Trennkolben 6 in der gleichen Blasformmaschine hergestellt, mittels deren auch die Ampullen hergestellt werden, und zwar aus einem Bereich des sich in der Blasform befindenden Materials, der für die Ampullenherstellung als Abfall anfallen würde. Der Trennkolben wird durch ein Hohlziehen dieses Abfalles, also in der gleichen Weise wie die Ampulle, geformt. Danach wird er aus dem ihn umgebenden Material herausgestanzt, wobei eine angeformte Dichtlippe am Trennkolben belassen werden kann, der Trennschnitt also im Abstand von der Mantelfläche des Trennkolbens geführt wird. Die so hergestellten Trennkolben werden dann in ein Magazin der Blasformmaschine transportiert, aus dem sie zum Einsetzen in die später geformten Ampullen entnommen werden.

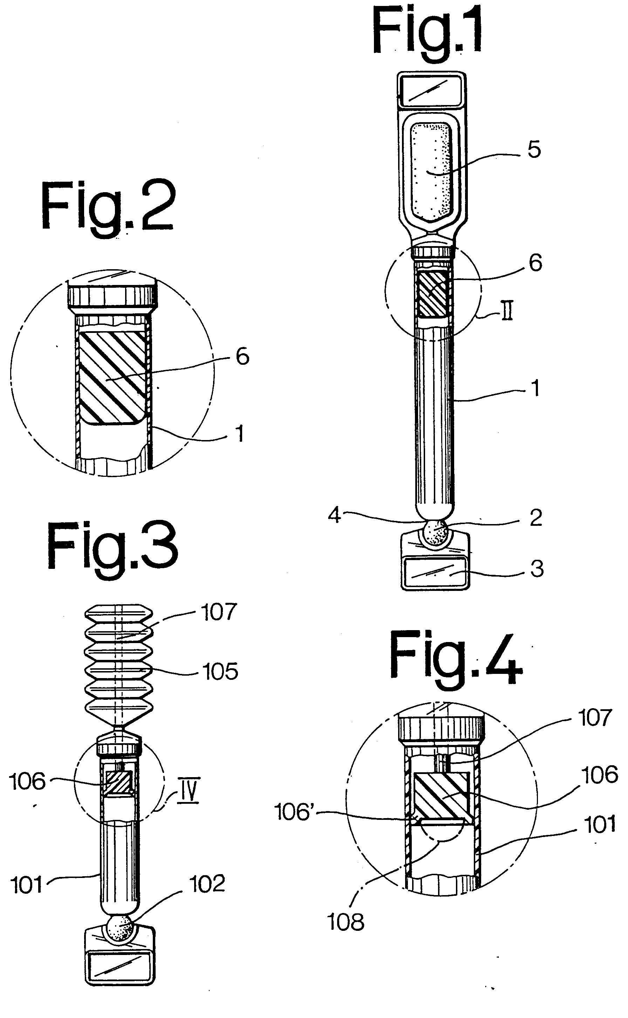

Bei dem in den Fig.3 und 4 dargestellten, zweiten Ausführungsbeispiel des erfindungsgemäßen Behältnisses handelt es sich ebenfalls um eine Ampulle, deren Ampullenkörper 101 und Brechverschluß 102 in gleicher Weise ausgebildet ist wie der Ampullenkörper 1 und der Brechverschluß 2 des ersten Ausführungsbeispiels. Ferner ist wie beim ersten Ausführungsbeispiel in den Ampullenkörper 101 ein Trennkolben 106 eingesetzt. Dieser Trennkolben 106 ist jedoch an einer einstückig mit ihm ausgebildeten Kolbenstange 107 vorgesehen, die koaxial zum Ampullenkörper 101 aus diesem über das dem Brechverschluß 102 abgekehrte Ende hinausragt. Dieser überstehende Abschnitt der Kolbenstange 107 liegt im Inneren eines Faltenbalges 105, der gleichachsig zum Ampullenkörper 101 an dessen dem Brechverschluß 102 abgekehrtes Ende angeformt ist, also wie die Kammer 5 des ersten Ausführungsbeispiels einstückig mit dem Ampullenkörper ausgebildet ist.

Wird der Faltenbalg 105 in axialer Richtung zusammengedrückt, dann wirkt auf den Trennkolben 106 nicht nur die aus dem Faltenbalg 105 verdrängte Luft, sondern auch die auf die Kolbenstange 107 ausgeübte Schubkraft. Deshalb kann der Trennkolben 106 problemlos auch dann gegen die Ausgabeöffnung des Ampullenkörpers 101 bewegt werden, wenn er mit einer gewissen Reibung an der Innenwand des Ampullenkörpers 101 anliegt. An das gegen die Ausgabeöffnung weisende Ende des Trennkolbens 106 ist deshalb eine in sich geschlossene, elastische Dichtlippe 106' angeformt, die an der Innenwand des Ampullenkörpers 101 anliegt. Da die Dichtlippe 106' gegen die Ausgabeöffnung hin über den Trennkolben 106 übersteht, wird sie umso stärker an die Innenwand des Ampullenkörpers 101 angedrückt, je größer der Druck ist, dem der im Ampullenkörper 101 enthaltene Stoff ausgesetzt ist. Die Dichtlippe 106' streift deshalb bei der Längsverschiebung des Trennkolbens 106 den an der Innenwand des Ampullenkörpers 101 haftenden Stoff praktisch vollständig ab. Sofern auch das halbkugelförmige Ende des Ampullenkörpers 101 möglichst weitgehend entleert werden soll, kann man an der von der Dichtlippe 106' berandeten Stirnseite des Trennkolbens 106 einen in Fig.4 mit einer strichpunktierten Linie dargestellten, halbkugelartigen Vorsprung 108 vorsehen.

Wie bei dem Ausführungsbeispiel gemäß den Fig.1 und 2 bestehen auch bei dem Ausführungsbeispiel gemäß den Fig.3 und 4 die beiden Teile, welche die Ampulle aufweist, vorzugsweise aus dem gleichen Material, beispielsweise aus Polyethylen oder Polypropylen. Im Bedarfsfall kann der Trennkolben 106 mit seiner Kolbenstange 107 auch aus Polyfluorethylen bestehen.

Besonders vorteilhaft ist auch bei diesem Ausführungsbeispiel die Herstellung in einer Blasformmaschine mit den im Zusammenhang mit dem ersten Ausführungsbeispiel geschilderten Verfahrensschritten.

Selbstverständlich könnte der Trennkolben 106 ohne die Dichtungslippe 106', also wie der Trennkolben 6 des ersten Ausführungsbeispiels, ausgebildet sein. Ebenso wäre es möglich, den Trennkolben 6 des ersten Ausführungsbeispiels mit einer elastischen Dichtlippe und/oder einem dem Vorsprung 108 entsprechenden, halbkugelförmigen Vorsprung zu versehen. Weiterhin könnte man statt einer Dichtlippe oder zusätzlich zu dieser ein Dichtelement in Form eines O-Ringes vorsehen, wodurch jedoch der Aufwand vergrößert werden würde, der ein Minimum erreicht, wenn die Ampulle nur aus zwei Teilen besteht und entsprechend dem erfindungsgemäßen Verfahren hergestellt wird.

## Patentansprüche

1. Behältnis aus Kunststoff für eine dosierbare Abgabe von fließfähigen, insbesondere pasteusen Stoffen, mit einer am einen Ende vorgesehenen Ausgabeöffnung und einer sich an das andere, offene Ende anschließenden und nur zum Inneren des Behältnisses hin offenen Kammer, deren Volumen durch eine manuelle Deformation reduzierbar ist und die einstückig mit dem Behältnis ausgebildet ist, wobei das Behältnis als ein Zylinder ausgebildet ist, wobei der den fließfähigen Stoff aufnehmende Innenraum des Zylinders vom Innenraum der Kammer durch einen im Zylinder von Hand längsverschiebbaren Trennkolben getrennt ist und wobei in der Kammer Luft bevorratet ist, die von Hand verdrängt den Trennkolben gegen die Ausgabeöffnung des Behältnisses drückt, dadurch gekennzeichnet, daß die Ausgabeöffnung des Behältnisses (1;101) mittels eines Brechverschlusses verschlossen ist und daß die Kammer (5) blockförmig ausgebildet und von dünnen Wänden begrenzt oder aus einem Balg (105) gebildet ist.

2. Behältnis nach Anspruch 1, dadurch gekennzeichnet, daß der Trennkolben (6; 106) ein aus Kunststoff bestehendes Einlegeteil ist.

3. Behältnis nach Anspruch 1 oder 2, dadurch gekennzeichnet, daß an den Trennkolben (106) wenigstens eine in sich geschlossene Dichtlippe (106') angeformt ist.

4. Behältnis nach einem der Ansprüche 1 bis 3, dadurch gekennzeichnet, daß der Trennkolben eine Ringnut aufweist, in der ein an der Innenwand des Zylinders anliegender O-Ring liegt.

5. Behältnis nach einem der Ansprüche 1 bis 4, dadurch gekennzeichnet, daß die Kammer als ein sich in Längsrichtung des Zylinders erstreckender Faltenbalg (105) ausgebildet ist.

6. Behältnis nach Anspruch 5, dadurch gekennzeichnet, daß der Faltenbalg (105) eine den Trennkolben (106) tragende, sich beim Zusammendrücken des Faltenbalges (105) gegen die Ausgabeöffnung hin verschiebender Kolbenstange (107) enthält.

7. Behältnis nach Anspruch 6, dadurch gekennzeichnet, daß die Kolbenstange (107) einstückig mit dem Trennkolben (106) ausgebildet ist.

8. Behältnis nach einem der Ansprüche 1 bis 4, dadurch gekennzeichnet, daß die Kammer (5) als ein quer zur Längsrichtung des Behältnisses zusammendrückbarer Balg ausgebildet ist.

9. Behältnis nach einem der Ansprüche 1 bis 4, dadurch gekennzeichnet, daß es an dem die Ausgabeöffnung bildenden Ende durch einen Brechverschluß (2; 102) verschlossen ist.

10. Verfahren zur Herstellung eines Behältnisses gemäß einem der Ansprüche 1 bis 9, dadurch gekennzeichnet, daß
a) das Behältnis im Blasformverfahren geformt und an seinem die Ausgabeöffnung bildenden Ende verschlossen wird,
b) in den vom Behältnis gebildeten Zylinder der im Behältnis aufzubewahrende, fließfähige Stoff eingefüllt wird,
c) danach der Trennkolben (6; 106) eingesetzt wird und
d) zum Schluß die Kammer (5; 105) im Blasformverfahren geformt und dabei das Behältnis verschlossen wird.

11. Verfahren nach Anspruch 10, dadurch gekennzeichnet, daß der Trennkolben (6;106) aus dem der Herstellung des Behältnisses dienenden Material in einem für die Formung des Behältnisses nicht benötigten Bereich durch Blasformen gebildet, danach von dem ihn umgebenden Material getrennt und danach der Blasformmaschine für ein Einsetzen in ein später geformtes Behältnis zugeführt wird.

## Claims

1. Plastics container for a metered delivery of flowable, in particular pasty substances, with a delivery opening provided at one end and a chamber adjoining the other, open end and which is open solely towards the inside of the container, the volume of which chamber can be reduced by manual deformation and which is constructed in one piece with the container, the container being constructed as a cylinder, the interior of the cylinder receiving the flowable substance being separated from the interior of the chamber by a separating piston which can be displaced longitudinally by hand in the cylinder and air being stored in the chamber, which compressed by hand, pushes the separating piston towards the delivery opening of the container, characterised in that the delivery opening of the container (1; 101) is closed by means of a breaking closure member and that the chamber (5) is constructed in the shape of a block and is defined by thin walls or is formed by bellows (105).

2. Container according to Claim 1, characterised in that the separating piston (6; 106) is an insertion part consisting of plastics material.

3. Container according to Claim 1 or 2, characterised in that integrally formed on the separating piston (106) is at least one closed sealing lip (106').

4. Container according to one of Claims 1 to 3, characterised in that the separating piston comprises an annular groove, in which lies an O-ring bearing against the inner wall of the cylinder.

5. Container according to one of Claims 1 to 4, characterised in that the chamber is constructed as bellows (105) extending in the longitudinal direction of the cylinder.

6. Container according to Claim 5, characterised in that the bellows (105) contain a piston rod (107) supporting the separating piston (106) and moving towards the delivery opening at the time of compression of the bellows (105).

7. Container according to Claim 6, characterised in that the piston rod (107) is constructed in one piece with the separating piston (106).

8. Container according to one of Claims 1 to 4, characterised in that the chamber (5) is constructed as bellows which can be compressed at right angles to the longitudinal direction of the container.

9. Container according to one of Claims 1 to 4, characterised in that at the end forming the delivery opening it is sealed by a breaking closure member (2; 102).

10. Method for the manufacture of a container according to one of Claims 1 to 9, characterised in that
a) the container is formed by the blow moulding method and is sealed at its end forming the delivery opening,
b) the flowable substance to be stored in the container is introduced into the cylinder formed by the container,
c) then the separating piston (6; 106) is inserted and
d) finally the chamber (5; 105) is formed by the blow moulding method and thus the container is sealed.

11. Method according to Claim 10, characterised in that the separating piston (6; 106) is formed by blow moulding from the material serving for the manufacture of the container, in a region not required for the formation of the container, then it is separated from the material surrounding it and then it is supplied to the blow moulding machine for insertion in a container formed at a later time.

## Revendications

1. Récipient en plastique pour une distribution dosable de substances fluides, en particulier pâteuses, comprenant un orifice de sortie prévu à l'une des extrémités et une chambre qui est ouverte uniquement vers l'intérieur du récipient, est reliée à l'autre extrémité ouverte, dont le volume peut être réduit par une déformation manuelle et qui est réalisée d'un seul tenant avec le récipient, le récipient étant conçu comme un cylindre, l'intérieur du cylindre logeant la substance fluide étant séparé de l'intérieur de la chambre par un piston de séparation déplaçable à la main dans le cylindre, dans le sens longitudinal et l'air stocké dans la chambre étant expulsé à la main et poussant le piston de séparation contre l'orifice de sortie du récipient, caractérisé en ce que l'orifice de sortie du récipient (1 ; 101) est fermé à l'aide d'une fermeture de sécurité et en ce que la chambre (5) est réalisée d'un seul tenant et est délimitée par des parois minces ou formée par un soufflet (105).

2. Récipient selon la revendication 1, caractérisé en ce que le piston de séparation (6 ; 106) est une pièce d'insertion en plastique.

3. Récipient selon la revendication 1 ou la revendication 2, caractérisé en ce que, au moins une lèvre d'étanchéité (106') fermée sur elle-même est moulée sur le piston de séparation (106).

4. Récipient selon l'une quelconque des revendications 1 à 3, caractérisé en ce que le piston de séparation présente une rainure annulaire dans laquelle se trouve un joint torique reposant contre la paroi intérieure du cylindre.

5. Récipient selon l'une quelconque des revendications 1 à 4, caractérisé en ce que la chambre est réalisée comme un soufflet (105) s'étendant dans le sens longitudinal du cylindre.

6. Récipient selon la revendication 5, caractérisé en ce que le soufflet (105) contient une tige de piston (107) portant le piston de séparation (106) et se déplaçant contre l'orifice de sortie lors de la compression du soufflet (105).

7. Récipient selon la revendication 6, caractérisé en ce que la tige de piston (107) est réalisée d'un seul tenant avec le piston de séparation (106).

8. Récipient selon l'une quelconque des revendications 1 à 4, caractérisé en ce que la chambre (5) est réalisée comme un soufflet compressible transversalement par rapport au sens longitudinal du récipient.

9. Récipient selon l'une quelconque des revendications 1 à 4, caractérisé en ce qu'il est fermé à l'extrémité formant l'orifice de sortie, par une fermeture de sécurité (2 ; 102).

10. Procédé de fabrication d'un récipient selon l'une quelconque des revendications 1 à 9, caractérisé en ce que
a) le récipient est moulé selon un procédé de moulage par soufflage et est fermé à son extrémité formant l'orifice de sortie,
b) la substance fluide devant être conservée le récipient est versée dans le cylindre formé par le récipient,
c) le piston de séparation (6 ; 106) est ensuite inséré et en ce que
d) pour finir, la chambre (5 ; 105) est moulée selon un procédé de moulage par soufflage et le récipient est fermé en même temps.

11. Procédé selon la revendication 10, caractérisé en ce que le piston de séparation (6 ; 106) est formé selon un moulage par soufflage, dans la matière servant à la fabrication du récipient, dans une zone non nécessaire au moulage du récipient, en ce qu'il est ensuite séparé de la matière qui l'entoure et en ce qu'il est ensuite amené à la machine de moulage par soufflage pour une insertion dans un récipient moulé ultérieurement.
